# EUROPEAN PATENT APPLICATION

(11) **EP 1 421 898 A1**
(43) Date of publication of application: **26.05.2004**
(21) Application number: 02025860.4
(22) Date of filing: 19.11.2002
(51) Int. Cl.: A61B 5/022

(54) **Method and device for the non-invasive measurement of the blood pressure**

(71) Applicant: Microlife Intellectual Property GmbH, 9435 Heerbrugg (CH)
(72) Inventor: Lin, Kin Yuan, Taipei, 114 (TW)
(74) Representative: Müller, Christoph Emanuel

(57) **Abstract**

A device for the non-invasive automatic measurement of a blood pressure is provided with a display adapted to display a two dimensional curve. A calculating arrangement (4) in the device (1) is adapted to display a curve (C) representing the pulse wave signal (W) measured with a pressure sensor (3) for measuring the pressure (P) inside the cuff (2) of the blood pressure monitor (1).

## Description

The invention relates to a device for the non-invasive measurement of the blood pressure and to a method for operating a blood pressure monitor. There are known a plurality of devices for automatically measuring the blood pressure of a patient. Such devices typically use an inflatable cuff which is wound around the patient's arm, e.g. around the upper arm or the wrist. The blood pressure may be e.g. detected by detection of Korotkoff sounds appearing and disappearing independence on a decreasing pressure within the cuff. A system which is commonly used today is the so-called oscillometric method. Blood pressure values are determined on the basis of a pulse wave signal representing the inside pressure within the cuff, when the cuff pressure is decreased, i.e. when the cuff is deflated. The pulse wave signal is generally measured with a pressure sensor arranged within a housing of the measuring device. One problem of such oscillometric measurement methods is, that errors in the calculation of the blood pressure may occur due to an inappropriate behaviour of the patient. For a proper measurement, the patient should be at a rest position and should not talk. In addition, physiologic incidents such as arrhythmia or tremor may lead to inaccurate results.

It is known in the art to use the pulse wave curve for the purpose of diagnosis by a medical person. US 6,045,510 e.g. discloses to print out graphs representing the pulse wave form on printer allowing a medical person to make a diagnosis. US 4,484,584 is directed to a device for measuring the blood pressure based on Korotkoff measurements. For this purpose, a graph may be displayed with a dot printer or on a display formed by lamps arranged in a matrix or by an electron tube. EP 365,614 is directed to a method where an information stream directed to data about the operation of the heart is displayed on a graphic display. Such data may be the cuff pressure as a function of time. US 6,394,959 discloses a continuous blood pressure monitor having a specific pressure pulse wave sensor and which is provided with an amplitude change curve displaying device. The pressing force of a sensor is displayed versus an amplitude of the pulse of the pressure pulse wave.

None of these documents allows to avoid the above-mentioned drawbacks of common oscillometric measurement devices.

It is therefore an object of the present invention to overcome the drawbacks of the prior art, especially to provide a device for the non-invasive, automatic oscillometric measurement of the blood pressure and a method for operating such a device which allows to avoid inaccurate measurement results e.g. based on arrhythmias, artefacts or the like.

These objects are solved with a device and a method according to the independent patent claims.

The device according to the invention is used for the automatic, non-invasive measurement of the blood pressure. The device is basically designed in a known manner and comprises an inflatable cuff, a pressure sensor and a calculating arrangement. The inflatable cuff is adapted to be wound around a patient's arm. The pressure sensor is used for measuring the pressure inside the cuff and for generating a pulse wave signal. The calculating arrangement is used for oscillometrically calculating at least one blood pressure value on the basis of the pulse wave signal. Methods for oscillometrically calculating the blood pressure values are known in the art as such. According to the invention, the device further comprises a display which is adapted to display a two dimensional curve. The calculating arrangement is adapted to display a curve representing the pulse wave signal on the display. This device allows to display the pulse wave signal on the display during the measurement. Artefacts or physiologic irregularities may be directly noticed by the user observing the curve on the display. If the user observes such irregularities, she or he may restart the measurement process. The inventors have found, that with such a display feature, errors occurring during oscilometric measurement may be greatly reduced as the user's awareness of inappropriate behaviour is enhanced.

According to a preferred embodiment of the invention, the cuff is adapted to be wound around the wrist of a patient. The risk of motion artefacts mostly appears in context with measurements made on the wrist of a patient. The risk of moving the wrist is much higher than the risk of moving the upper arm. The present invention therefore is especially suitable for such wrist type blood pressure monitors. Such devices are widely used as home measurement devices, because it is much easier to apply a cuff around a wrist than around the upper arm. Especially, elderly persons might have difficulties to correctly apply a cuff around the upper arm without assistance from another person.

According to a further preferred embodiment, the cuff is directly connected to a housing. In this case, the display is preferably arranged on this housing. With such a so-called wrist watch type blood pressure monitor, the maximum comfort is achieved for the user. The cuff may be easily applied around the wrist. There is no tubing which might render the use of the device uncomfortable for the user. The present invention helps to avoid the drawbacks of such known wrist type blood pressure monitors, namely the risk of motion artefacts due to the application site around the wrist. According to a further preferred embodiment of the invention, the display may be formed as a dot matrix. The dot matrix has several advantages. Especially, it may be used both to display a curve representing the pulse wave signal and to display user instructions, additional user information or the blood pressure values once the measurement has been completed.

The present invention is typically adapted to calculate the systolic and the diastolic blood pressure value. Additional measurement parameters such as the mean arterial blood pressure or the pulse rate may be calculated and displayed.

The device according to the invention may be further provided with a display having text display field. The text display field may be used to display instructions to the user. Such instructions typically may consist in short information such as "do not talk" or "do not move". These instructions may be used to limit the risk of measurement errors due to an incorrect behaviour of the user. According to a further preferred embodiment, the calculating arrangement may be adapted to detect the nature of an artefact or another pulse wave alteration on the basis of the pulse wave signal. Appropriate instructions to the user may be displayed on the text display field in accordance with the nature of the detected pulse wave alteration or disturbance. Such determination may typically be made by means of comparing the pulse wave signal with known patterns of correct and altered signals, which may be stored in a memory within the device.

The method according to the present invention is directed to the operation of a blood pressure monitor. According to the method of the invention, the pressure in an inflatable cuff is measured while the pressure within the cuff is decreased. By means of this measurement, a pulse wave signal representing the pressure within the cuff is generated. This pulse wave signal is subsequently displayed on a display in the form of a two dimensional curve. In a further step, at least one blood pressure value is oscillometrically determined on the basis of the pulse wave signal.

The method according to the present invention is preferably used in context with an inflatable cuff, which is wound around the patient's wrist. The curve may be displayed on a display, which is arranged in a housing, which is mechanically connected directly to the cuff. The curve may be displayed on a dot matrix.

According to a further preferred embodiment of the invention, instructions to the user may be displayed on a text field within the display. In addition, the content of such instructions may be selected on the basis of the type of alterations of the pulse wave signal. The type of alterations may be determined in the calculating arrangement.

The foregoing and other advantages of the invention will become apparent upon reading the following detailed description and upon reference to the drawings in which:
Figure 1 shows a three dimensional representation of a blood pressure monitor.
Figure 2 shows a schematic representation of a blood pressure monitor according to the invention.
Figures 3a and 3b show displays which may be typically made with a device according to the present invention.
Figures 4a to 4e show different types of pulse wave signal curves displayed on a display in a device according to the invention.

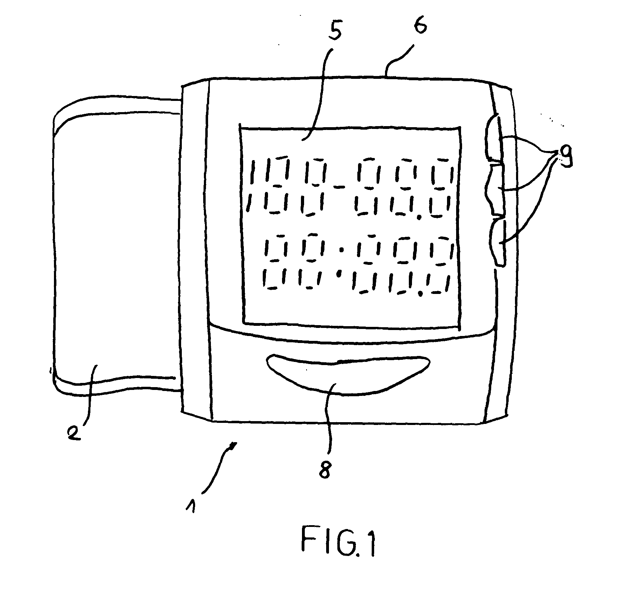

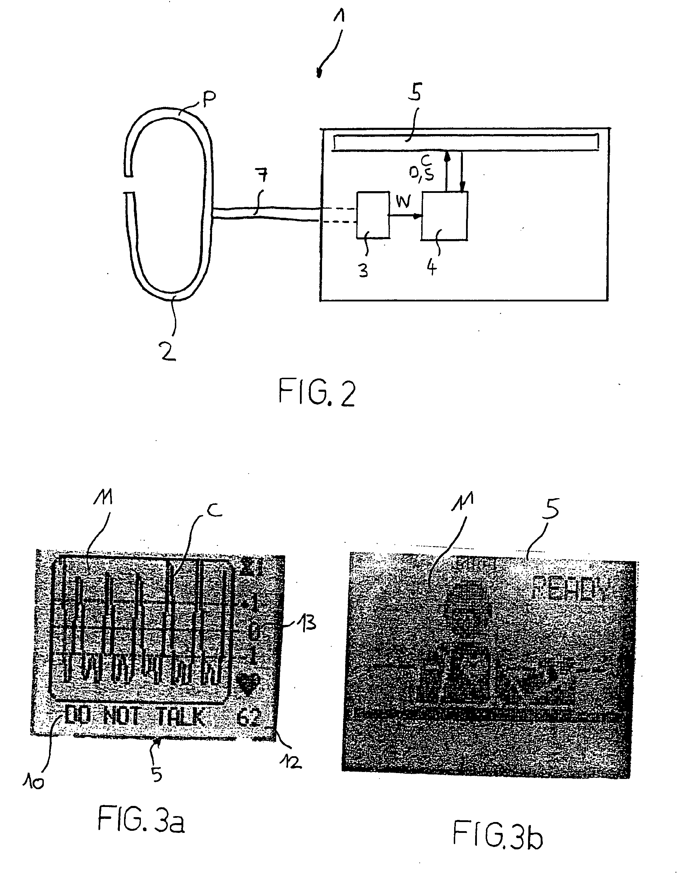

Figure 1 shows a blood pressure monitor 1 of the wrist watch type. The blood pressure monitor 1 comprises a housing 6 to which an inflatable cuff 2 is mechanically connected in a known manner. A display 5 in the form of a dot matrix is arranged within the housing 6. The blood pressure monitor 1 is provided with an on-off button 8 and with operation buttons 9 allowing to enter specific user instructions. The blood pressure monitor 1 may be typically operated by means of selections from a menu displayed on the display 5. The internal design of the blood pressure monitor 1 is shown in more detail in figure 2. The pressure P within the cuff 2 is measured with a pressure sensor 3 connected to the cuff 2 by means of a tube 7 in a known manner. The pulse sensor 3 produces a pulse wave signal W which is supplied to a calculating arrangement 4. The calculating arrangement 4 in a known manner oscillometrically calculates the values of the systolic blood pressure S and the diastolic blood pressure D on the basis of the pulse wave signal W provided by the sensor 3. In addition, the calculating arrangement 4 is adapted to display a two dimensional curve C showing the pulse wave signal W in dependence of the time on the display 5. If the display is formed as a touch screen, instructions may be transmitted from the display to the calculating arrangement, as indicated by an arrow. According to figure 1, the cuff 2 is directly connected to the housing 6 of the blood pressure monitor 1. It is also possible (as indicated in figure 2) to connect the cuff 2 only by means of a tube 7 to the housing of the blood pressure monitor 1.

Figure 3a shows a typical representation of the curve C showing the pulse wave signal. The curve C is displayed on a graphic display field 11 on the display 5. In addition, the display 5 comprises a text display field 10, a pulse information display field 12 and a legend field 13. Text messages to the users such as "do not talk" may be displayed in the text display field 10. Pulse information such as the current pulse rate may be displayed in the pulse display field 12. A legend related to the amplitude of the curve C of the pulse wave signal W may be displayed in the legend display field 13. During the measurement of the blood pressure, the user may observe the curve C. Alterations or unexpected behaviour of the form may indicate that the user should not rely on the measurement result and restart the measurement procedure.

The display 5 according to the invention may also be used to display additional information, such as e.g. graphically instructing the user to hold the arm in a manner such that the blood pressure monitor 1 is arranged about at the level of the heart.. Such a pictogram is shown in figure 3b. The advantage of a display 5 in the form of a dot matrix is, that almost any type of information or representation may be displayed by appropriate programming of the calculating arrangement 4.

The calculating arrangement 4 is a common microprocessor. The display 5 is a common dot matrix. Those skilled in the art can easily find appropriate microprocessors and displays for the purposes of the present invention.

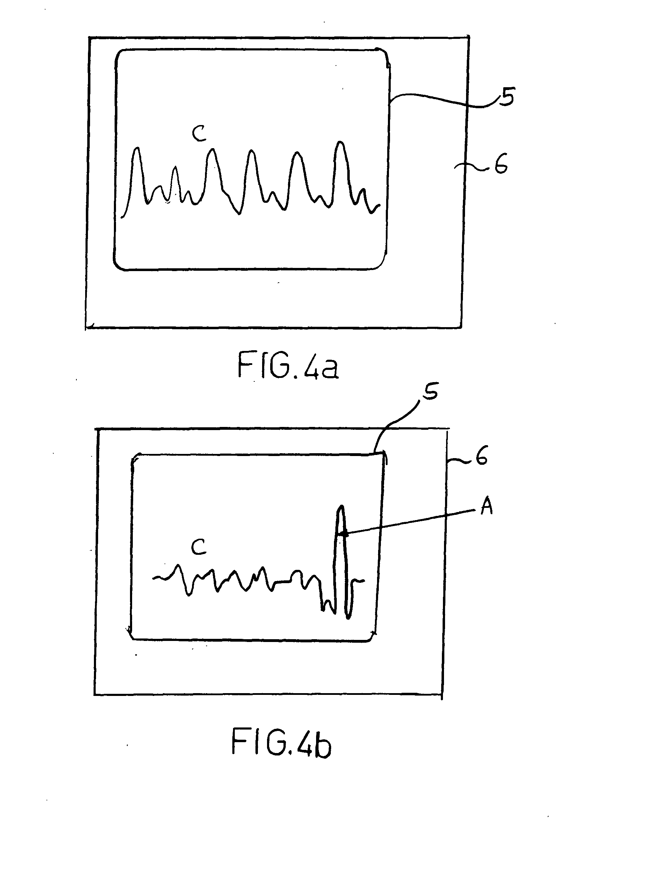

Figures 4a to 4e show different types of pulse wave signals displayed in curves C on the display 5 arranged in the housing 6.

Figure 4a shows a curve C of a typical pulse wave signal. This signal is basically unaltered and can be used for a proper measurement of the blood pressure. The pulses are regularly spaced. They have a substantially constant amplitude. The user knows that he may rely on the measurement result if the curve C representing the pulse wave signal W has regular pulses with a substantially constant amplitude.

Figure 4b shows a curve C which has been altered because of a motion of the user. The motion artefact A is represented by an amplitude which is much higher than the amplitudes of the rest of the curve C. A user seeing that the curve C has a peak, knows that he should not rely on a measurement result and that the measurement should be restarted. In addition, the type of alteration may give the user an indication of the reason why an error occurred. A user seeing a curve as represented in figure 4b will know that he should try to be in a rest position before the measurement is started. Appropriate information including typical samples of altered pulse wave curves may be provided to the user in the operation manual.

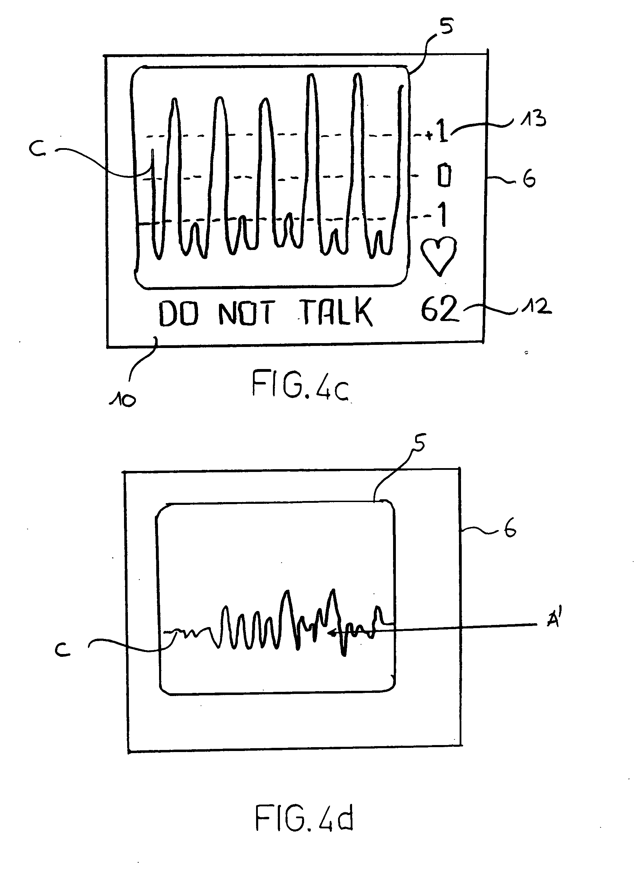

Figure 4c shows another representation of a curve C without any alterations. In addition to the curve C the user is given instructions on how to behave during the measurement of the blood pressure. Such instructions may typically tell the user not to move or not to talk. It is possible to display such messages on the basis of previously measured pulse wave signals. On the basis of such signals, the calculating arrangement 4 may determine the reason of measurement errors and instruct the user to change his or her behaviour in order to avoid such errors in the next measurement.

Figure 4d shows a curve C of a pulse wave signal W, which has been altered because of tremor. A tremor artefact is shown in the area A' of the curve C.

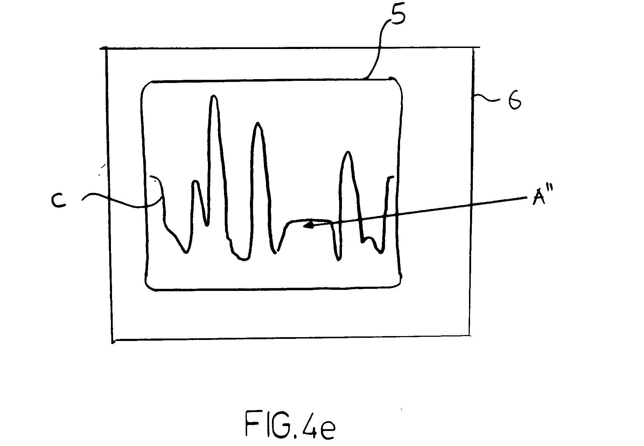

Figure 4e shows a curve C of pulse wave signal which has been made when an arrhythmia has occurred. A pulse is missing in the area A" on the curve C.

The curve C is typically displayed such that the separate pulses may be discerned from each other. Typically, 6 to 8 pulses may be displayed. This means, that the scale of the display in the vertical direction (representing the time) will correspond to about 4 to 10 seconds. During the measurement, the pulse wave signal of a preceding period of time, e.g. of the last 4 to 10 seconds will be shown. A value corresponding to the current value of the pressure within the cuff is always shown on the right hand side of the display 5.

As the pulse wave signal W is typically stored in a memory within the blood pressure monitor, the user may review the pulse wave signal of the complete measurement once the measurement has been terminated. It will be appreciated, that the amplitude of the signal is decreasing in view of the deflation of the cuff. Such a decrease is, however, not clearly discernible in the short period of time. The difference between figure 4a and 4c, both showing a regular pulse wave signal is e.g. due to the fact that the curve at different periods of time during the complete measurement is shown.

The device may be programmed such that always the same number of pulses, e.g. 6 pulses are shown; irrespective of the pulse rate. In this case, the scale of the horizontal axis is variable and determined on the basis of the pulse rate.

## Claims

1. A device (1) for the non-invasive automatic measurement of the blood pressure comprising
- an inflatable cuff (2) adapted to be wound around a patient's arm
- a pressure sensor (3) for measuring the pressure (P) inside the cuff and for generating a pulse wave signal (W)
- a calculating arrangement (4) for oscillometrically calculating at least one blood pressure value (D, S) on the basis of said pulse wave signal (W)
wherein said device (1) comprises a display (5) adapted to display a two dimensional curve (C) and wherein said calculating arrangement (4) is adapted to display a curve (C) representing said pulse wave signal (W), on said display.

2. A device according to claim 1, wherein said cuff (2) is adapted to be wound around the wrist of a patient.

3. A device according to one of the claims 1 or 2, wherein a housing (6) is mechanically connected directly to the cuff (2) and wherein said display (5) is arranged within the housing.

4. A device according to one of the claims 1 to 3, wherein said display (5) is formed as a dot matrix.

5. A device according to one of the claims 1 to 4, wherein the calculating arrangement (4) is adapted to calculate at least the systolic and the diastolic blood pressure value (D, S).

6. A device according to one of the claims 1 to 5, wherein the display (5) is provided with a text display field (10) on which instructions to the user may be displayed.

7. A device according to claim 6, wherein the calculating arrangement (4) is adapted to detect the nature of a pulse wave alteration and to display an appropriate instruction to the user for avoiding such alteration.

8. A method for operating a blood pressure monitor, comprising the steps of
- measuring the pressure (P) in an inflatable cuff (2) while decreasing the pressure (P) within the cuff (2)
- generating a pulse wave signal (W) representing the pressure within the cuff (2),
- displaying said pulse wave signal (W) on a display in the form of a two dimensional curve (C)
- oscillometrically determining at least one blood pressure value (D, S) on the basis of said pulse wave signal (W).

9. A method according to claim 8, wherein the pressure (P) is measured in an inflatable cuff (2) which is wound around a patient's wrist.

10. A method according to one of the claims 8 or 9, wherein said curve (C) is displayed on a display arranged in a housing (6) mechanically connected directly to the cuff (2).

11. A method according to claim 10, wherein said curve (C) is displayed on a dot matrix (5).

12. A method according to one of the claims 8 to 11, wherein a text message is displayed on said display (5).

13. A method according to claim 12, wherein the pulse wave (W) is analysed in the calculating arrangement (4) for detecting the nature of a possible pulse wave signal alteration and wherein an appropriate information to the user is displayed on the display field (5) if a pulse wave signal alteration has been detected.
